# EUROPÄISCHE PATENTSCHRIFT

(11) **EP 1 228 271 B1**
(45) Veröffentlichungstag und Bekanntmachung des Hinweises auf die Patenterteilung: **26.05.2004**
(21) Anmeldenummer: 00964252.1
(22) Anmeldetag: 29.09.2000
(51) Int. Cl.: D04H 13/00, D04H 1/42

(54) **VERBUNDWERKSTOFF ZUR BILDUNG EINER KÖRPERZUGEWANDTEN LAGE BEI EINEM HYGIENEARTIKEL SOWIE HYGIENEARTIKEL**
COMPOSITE MATERIAL FOR PRODUCING A LAYER OF A HYGIENIC ARTICLE THAT COMES INTO PHYSICAL CONTACT WITH THE BODY AND A CORRESPONDING HYGIENIC ARTICLE
MATERIAU COMPOSITE SERVANT A FORMER UNE COUCHE, ORIENTEE VERS LE CORPS, D'UN ARTICLE D'HYGIENE, ET ARTICLE D'HYGIENE AINSI PRODUIT

(30) Priorität: 02.10.1999 DE 19947582
(43) Veröffentlichungstag der Anmeldung: 07.08.2002
(73) Patentinhaber: Paul Hartmann Aktiengesellschaft, 89522 Heidenheim (DE)
(72) Erfinder: MANGOLD, Rainer, 89542 Herbrechtingen (DE); MALOWANIEC, Krzysztof-Daniel, 89522 Heidenheim (DE); ECKEL, Petra, 85640 Putzbrunn (DE)
(74) Vertreter: Friz, Oliver
(86) Internationale Anmeldenummer: PCT/EP2000/009537
(87) Internationale Veröffentlichungsnummer: WO 2001/025523

(56) Entgegenhaltungen:
- EP-A- 0 780 108
- EP-A- 0 792 629
- WO-A-96/12460
- US-A- 5 143 779

## Beschreibung

Die Erfindung betrifft einen Verbundwerkstoff zur Bildung einer körperzugewandten Lage bei einem absorbierenden Hygieneartikel zum einmaligen Gebrauch, aus wenigstens zwei durch thermische Einwirkung miteinander verbundenen Vlieslagen.

Derartige mehrschichtige Verbundwerkstoffe sowie Hygieneartikel mit einer körperzugewandten, üblicherweise als Topsheet bezeichneten Lage aus einem solchen Verbundwerkstoff, sind bekannt. Die körperzugewandte Lage überfängt üblicherweise einen darunter, also körperabgewandt angeordneten Speicherkörper, in dem die vom Benutzer abgegebene Körperflüssigkeit gespeichert werden soll, was bei modernen Hygieneartikeln vornehmlich durch superabsorbierende Materialien innerhalb des Speicherkörpers erreicht wird.

An Verbundwerkstoffe der in Rede stehenden Art werden hohe Anforderungen im Hinblick auf eine schnelle Flüssigkeitsaufnahmefähigkeit auch bei wiederholter schwallartiger Einnässung gestellt. Es soll verhindert werden, daß sich auftreffende Flüssigkeit an der Oberfläche ansammelt (pooling) und zu den Seiten hin ausweicht, sondern die auftreffende Flüssigkeit soll von der körperzugewandten Lage absorbiert und in Richtung auf den Speicherkörper weitergeleitet werden. Auch eine Verteilerwirkung innerhalb der Schicht ist erwünscht. Des weiteren soll das Rücknässungsverhalten des Verbundwerkstoffs, insbesondere bei Druckbeaufschlagung, wie sie beispielsweise durch das Körpergewicht eines Benutzers beim Sitzen hervorgerufen werden kann, so gering wie möglich sein. Der Verbundwerkstoff soll also eine Abstandshalterfunktion zwischen dem absorbierenden Speicherkörper eines Hygieneartikels und der Hautoberfläche ausüben.

Diejenige Oberfläche, welche mit der Haut eines Benutzers in Berührung steht, soll als weich und angenehm empfunden werden, was sich durch die Verwendung sehr feiner Fasern erreichen läßt. Die Verwendung sehr feiner Fasern wiederum steht im Widerspruch zu dem Bestreben, ein möglichst bauschelastisches Verhalten des Verbundwerkstoffs zu erreichen. Hierunter versteht man die Fähigkeit des Verbundwerkstoffs eine hohe Rückstellkraft gegenüber dem Einwirken von äußeren Druckkräften entgegenzusetzen bzw. nach Einnässung und/oder Zusammendrücken wieder wenigstens nahezu in den Ausgangszustand zurückzukehren.

US 5,257,982 beschreibt einen Verbundwerkstoff zur Bildung einer körperzugewandten Lage bei einem absorbierenden Hygieneartikel, der aus wenigstens zwei Vlieslagen gebildet ist, wobei jede Vlieslage eine erste körperzugewandte Schicht und eine zweite darunter, also körperabgewandt, angeordnete Schicht aufweist. Die Faserstärke der körperabgewandt angeordneten zweiten Schicht ist geringer als die Faserstärke der körperzugewandt angeordneten ersten Schicht. Die Druckschrift lehrt, Schichten großer Faserstärke körperzugewandt und Schichten niedrigerer Faserstärke körperabgewandt anzuordnen. Bei mehreren Schichten werden diese in schrittweise abnehmender Faserstärke angeordnet. Die Schichten können thermoplastische Fasern aus Polyamiden, Polyolefinen, Polypropylen umfassen, auch Fasern aus niedrig schmelzendem Polyester sind erwähnt.

EP 0 372 572 A2 beschreibt Polyester-Bindefasern zur thermischen Verfestigung von Faservliesen, die einen verhältnismäßig niedrigen Schmelzpunkt zwischen 160° bis 220° Celsius aufweisen. Weitere Hinweise lassen sich dieser Druckschrift nicht entnehmen.

Aus EP-A-0 859 883 B1 ist eine Vielzahl von Verbundwerkstoffen mit wenigstens zwei Vlieslagen bekannt, welche auch zur Bildung einer körperzugewandten Lage bei einem Hygieneartikel verwendet werden können. Diese Druckschrift befaßt sich mit der Verbesserung der Flüssigkeitsaufnahme- und -verteilercharakteristik des Verbundwerkstoffs als Topsheet bei einem Hygieneartikel, insbesondere sollen Durchtrittszeiten und Rücknässungsverhalten verbessert werden. Es werden zwei- oder dreischichtige Verbundwerkstoffe unterschiedlichster Schichtenzusammensetzung offenbart, wobei Mischungen synthetischer Bindefasern und matrixbildender Fasern zum Einsatz kommen.

Der vorliegenden Erfindung liegt die Aufgabe zugrunde, einen Verbundwerkstoff der eingangs genannten Art unter Berücksichtigung der vorstehend genannten generellen Anforderungen an ein Topsheet-Material weiter zu verbessern, und zwar im Hinblick auf die Festigkeit des Faserverbunds, also zwischen den Fasern einer Vlieslage aber auch zwischen den wenigstens zwei Vlieslagen, und im Hinblick auf die Neigung des Verbundwerkstoffs, bei der Handhabung Partikel (Fasern oder Faserteile) in die Umgebung abzugeben (Stauben). Es wurde nämlich festgestellt, daß Verbundwerkstoffe auf Vliesbasis bei der Handhabung und in besonderem Maße bei der Handhabung in schnell-laufenden Herstellungs- und Verarbeitungsmaschinen, z.B. solchen zur Herstellung von absorbierenden Hygieneartikeln, eine sehr starke Staubentwicklung mit sich bringen. Diesem weiteren Problem soll mit der vorliegenden Erfindung begegnet werden und damit die Verarbeitbarkeit von Verbundwerkstoffen aus Vlieslagen verbessert werden. Ferner soll ein Nachaußendringen der oft körnigen superabsorbierenden Polymermaterialien aus dem Speicherkörper durch die körperzugewandte Lage hindurch verhindert werden.

Diese Aufgabe wird durch einen Verbundwerkstoff aus wenigstens zwei durch thermische Einwirkung miteinander verbundenen Vlieslagen gelöst,
wobei die obere körperzugewandte Lage aus einer Mischung von Monokomponentenfasern und Bikomponentenfasern gebildet ist und der Anteil der Bikomponentenfasern 30 bis 70 Gew.-% der oberen Lage beträgt, und wobei die Feinheit der Fasern der oberen Lage höchstens 3,5 dtex beträgt, und
wobei die untere Lage zu wenigstens 40 Gew.-% Bikomponentenfasern umfaßt, deren höherschmelzende Komponente von PES (Polyester) gebildet ist und deren niedrigerschmelzende Komponente einen niedrigeren Schmelzpunkt aufweist als der der Monokomponentenfasern der oberen Lage, und wobei die Feinheit der Bikomponentenfasern der unteren Lage zwischen 4 und 10 dtex beträgt.

Die Bikomponentenfasern, deren niedrigerschmelzende Komponente durch thermische Einwirkung ein Bindemittel bildet, führen zu einer Verbesserung der inneren Festigkeit, d. h. des Zusammenhalts der Fasern untereinander aber auch der Fasern zwischen den Schichten. Dadurch, daß in der körperzugewandten oberen Lage außer Bikomponentenfasern auch höherschmelzende, sehr feine, also sehr dünne Monokomponentenfasern vorgesehen sind, wird die obere Lage als weich und angenehm empfunden. Ein Anteil der Bikomponentenfasern von 30 bis 70 Gew.-% hat sich als zielführend erwiesen. Werden zu wenig feine Monokomponentenfasern in der oberen Lage eingesetzt, die von der thermischen Einwirkung unberührt bleiben, so würde die Lage als zu hart empfunden werden. Werden zu wenig Bikomponentenfasern eingesetzt, die durch die thermische Einwirkung zur Verbindung der Fasern untereinander führen, so ist der Verbund innerhalb der Lage nicht hinreichend. Durch die Wahl der Feinheit der Fasern in der oberen Vlieslage von höchstens 3,5 dtex wird aber nicht nur dem Aspekt eines angenehmen Traggefühls Rechnung getragen, sondern es wird sehr wirkungsvoll ein weiteres Problem gelöst. Es wird hierdurch ein Nachaußendringen körnigen superabsorbierenden Partikelmaterials an die Oberfläche des Hygieneartikels verhindert. Es kann daher auf zusätzliche Hüllschichten des die superabsorbierenden Materialien enthaltenden Speicherkörpers, in Form von dünnen papierartigen Lagen oder dergleichen verzichtet werden.

Mit der Erfindung wurde erkannt, dass die oben erwähnte Staubproblematik auf einem Zielkonflikt beruht. Im Faserverbund soll die zumindestens eine untere Lage vor allem ein ausreichendes Hohlraumvolumen zur Verfügung stellen, um eine schnelle Flüssigkeitsaufnahme zu gewährleisten und als Abstandshalter zwischen dem (nassen) Speichersaugkörper und der Windelinnenseite fungieren. Dieses wird zum einen durch den Einsatz relativ steifer, bauschelastischer Fasern erreicht, zum anderen dadurch, dass die untere Lage bei der thermischen Verfestigung keine oder nur eine sehr geringe Komprimierung erfährt. Diese Umstände haben zur Folge, dass bei der thermischen Verfestigung relativ wenig Fasern miteinander in Kontakt kommen und damit relativ wenig Zwischenfaserverbindungen durch die thermische Einwirkung ausgebildet werden. Hieraus resultiert bei der Weiterverarbeitung des Verbundwerkstoffes die Staubproblematik. Unzureichend gebundene Fasern und Faserteile neigen dazu, sich aus dem Faserverbund zu lösen.

Durch die Wahl einer speziellen Bikomponentenfaser mit PES (Polyester) (oder mit einem PES im Hinblick auf das bauschelastische Verhalten solcher Bikomponentenfasern gleichkommenden Polymer) als höherschmelzende Komponente zu einem Anteil von mindestens 40 % bei gleichzeitiger Wahl einer Faserstärke zwischen 4 und 10 dtex wird sowohl eine ausgezeichnete Steifheit bzw. Bauschelastizität (resiliency) des Verbundwerkstoffs als auch eine hervorragende Bindung der Fasern untereinander erreicht.

Der PES-Kern dieser Bikomponentenfaser sorgt für eine ausreichende Steifheit der Faser und damit für die notwendige Bauschelastizität und die Aufrechterhaltung eines großen Hohlraümvolumens. Werden zu wenig Bikomponentenfasern mit PES (Polyester) als hochschmelzender Komponente eingesetzt, so ist die untere Lage entweder zu wenig bauschelastisch (falls eine andere Bikomponentenfaser eingesetzt wird, z. B. PP/PE (Polypropylen/Polyäthylen)-Bikomponentenfaser), oder die Fasern der unteren Lage sind nach der Thermofusion nicht hinreichend miteinander verbunden (falls eine bauschelastische Monokomponentenfaser als zusätzliche Faserkomponente eingesetzt wird). Letztere Konstellation würde bei der Weiterverarbeitung des Verbundwerkstoffes die oben beschriebene Staubproblematik fördern.

Die eingangs zitierte EP-A-0 859 883 B1 erwähnt zwar eine Vielzahl von Zusammensetzungen der einzelnen Schichten des Vliesverbundwerkstoffs. Nur einige wenige Ausführungsbeispiele umfassen Bikomponentenfasern mit PES (Polyester) als höherschmelzender Komponente. Die Schichtzusammensetzungen weichen jedoch in vielen anderen Parametern von der hier beanspruchten Zusammensetzung ab. Die Vorteilhaftigkeit von derartigen Bikomponentenfasern in der beanspruchten Zusammensetzung der Schichten wurde weder erkannt noch nahegelegt.

In vorteilhafter Weiterbildung der Erfindung weist die obere körperzugewandte Lage ein durch Kalandrieren erzeugtes Prägemuster auf, wobei der Anteil der geprägten Oberfläche 5 bis 30 %, vorzugsweise 15 bis 25 %, der Gesamtfläche des Verbundwerkstoffs beträgt. Durch die Kalandrierung wird die Festigkeit innerhalb des Verbundwerkstoffs weiter erhöht. Es wird auch die Dichtheit in Bezug auf ein ungewolltes und daher zu verhinderndes Austreten von superabsorbierenden Partikelmaterialien verbessert.

Es hat sich des weiteren als vorteilhaft erwiesen, wenn die obere Lage mit einem Flächengewicht von 10 bis 30, vorzugsweise von 15 - 20 g/m² ausgebildet wird. Die Flüssigkeitsaufnahmerate ist bei einem solchen Flächengewicht hinreichend und die Lage ist dennoch kostengünstig herstellbar.

Dadurch, daß die Fasern der oberen Lage hydrophilisiert sind, wird die Flüssigkeitsaufnahmerate weiter positiv beeinflußt.

In Weiterbildung der Erfindung umfaßt die untere Lage zu wenigstens 60 Gew.-%, vorzugsweise zu wenigstens 80 Gew.-% Bikomponentenfasern, deren höherschmelzende Komponente von PES (Polyester) gebildet ist. Ganz besonders bevorzugtermaßen besteht die untere Lage zu 100 % aus solchen Bikomponentenfasern. Je höher der Anteil dieser erfindungsgemäß gewählten Bikomponentenfasern in der unteren Lage des Vliesverbundwerkstoffs ist, desto bauschelastischer (resilient) erweist sich der Verbundwerkstoff bei gleichzeitig höherem Zwischenfaserbindungspotential.

Bikomponentenfasern können in bekannter Weise als Seite-an-Seite-Faser, als Kern/Mantel- oder auch als Matrix-Faser mit fibrillenartig eingelagerter niedrigerschmelzender Komponente hergestellt sein. Die Bikomponentenfaser mit PES (Polyester) als höherschmelzender Komponente ist vorzugsweise als Kern/Mantel-Faser mit einem zur Längsmittelrichtung der Faser exzentrisch angeordneten Kern ausgebildet. Die Stärke der Kern/Mantel-Bikomponentenfasern beträgt in Weiterbildung der Erfindung 5 bis 8 dtex und nach einer besonders bevorzugten Ausführungsform 6 bis 7 dtex.

Nach einer weiteren vorteilhaften Ausführungsform der Erfindung ist die niedrigerschmelzende Komponente der zu wenigstens 40 Gew.-% in der unteren Lage vorhandenen Bikomponentenfaser von PE (Polyäthylen) gebildet. Gerade die Kombination von PES als höherschmelzender Komponente und PE als niedrigerschmelzenden Komponente hat sich als vorteilhaft erwiesen, da solchenfalls die Verschweißbarkeit mit anderen Komponenten von absorbierenden Hygieneartikeln, beispielsweise dem üblicherweise aus PP oder PE gebildeten Backsheet, erleichtert wird.

Der vorliegenden Erfindung liegt die weitere Aufgabe zugrunde, einen Hygieneartikel mit einer flüssigkeitsdichten, im Gebrauch körperabgewandten Schicht, einem Speicherkörper und einer flüssigkeitsdurchlässigen auf der körperzugewandten Seite des Speicherkörpers vorgesehenen Schicht zu verbessern, und zwar sowohl im Hinblick auf Flüssigkeitsaufnahme und - Verteilereigenschaften und ein geringes Rücknässungsverhalten als auch im Hinblick auf die eingangs erwähnte Staubproblematik und auch die Barrierewirkung der körperabgewandten Schicht, um ein Austreten von superabsorbierenden Partikelmaterialien zur Oberfläche des Hygieneartikels zu verhindern.

Diese Aufgabe wird durch einen Hygieneartikel gelöst, wobei der Speicherkörper eine Lage aus intravernetzten Zellulosefasern mit einem Flüssigkeitsretentionswert zwischen 0,6 und 0,9 g_{F1}/g_{Faser} umfaßt, wobei die Lage intravernetzter Zellulosefasern zu 8 - 15 Gew.-% superabsorbierende Polymermaterialien enthält, wobei die flüssigkeitsdurchlässige auf der körperzugewandten Seite des Speicherkörpers vorgesehene Schicht ihrerseits wenigstens zweilagig ist und die obere dieser Lagen aus Fasern mit einer Feinheit von höchstens 3,5 dtex besteht, während die untere dieser Lagen Bikomponentenfasern mit einer Feinheit zwischen 4 und 10 dtex umfaßt, deren höherschmelzende Komponente von PES gebildet ist.

Das Flüssigkeitsrückhaltevermögen der vernetzten und nicht vernetzten natürlichen Zellstofffasern wird durch den folgenden Zentrifugentest durch Angabe des vorstehend erwähnten Flüssigkeitsretentionswerts bestimmt. Eine Schicht aus zu untersuchenden Zellstofffasern wird im trockenen Zustand gewogen, um deren Masse in Gramm zu ermitteln. Die Prüflinge werden dann 30 Minuten lang vollständig in einer einprozentigen Natriumchlorid-Lösung von demineralisiertem Wasser als Prüflösung eingetaucht und anschließend 4 Minuten bei 276-facher Erdbeschleunigung geschleudert. Danach werden die Prüflinge wiederum gewogen, um die Masse einschließlich der gebundenen Flüssigkeit zu bestimmen. Die Masse der aufgenommenen oder gebundenen Flüssigkeit ergibt sich daher aus der Differenz der nach dem Schleudern bestimmten Masse und der Trockenmasse des zu untersuchenden Fasermaterials. Dividiert man diese Differenz durch die Trockenmasse, so erhält man den Retentionswert in g_{F1}/g_{Faser}.

Dadurch, daß der Speicherkörper eine Lage intravernetzter Zellulosefasern mit darin enthaltenen 8 bis 15 Gew.-% dieser Lage an superabsorbierenden Polymermaterialien aufweist, wird einerseits ein Kollabieren des Speicherkörpers bei Flüssigkeitsbeaufschlagung verhindert, da intravernetzte Zellulosefasern bei Flüssigkeitsbeaufschlagung sich eher weiten als in sich zusammenfallen, was an sich bekannt und in modernen Hygieneartikeln genutzt wird. Andererseits wird durch den erfindungsgemäß hohen Anteil an superabsorbierenden Polymermaterialien in dieser vorzugsweise körperzugewandten Lage des Speicherkörpers darin verbliebene Flüssigkeit gebunden. Das Rücknässungsverhalten des Speicherkörpers und damit des Hygieneartikels wird daher entscheidend verbessert, da auch bei Druckbeaufschlagung, etwa durch das Körpergewicht eines Benutzers des Hygieneartikels, in dieser Lage noch vorhandene Restflüssigkeit nicht mehr zur Hautoberfläche des Benutzers gelangen kann, da sie durch die superabsorbierenden Materialien in dieser Lage ausreichend gebunden ist.

Durch die weitere Ausbildung der flüssigkeitsdurchlässigen, auf der körperzugewandten Seite des Speicherkörpers vorgesehen wenigstens zweilagigen Schicht, also dem Topsheet des Hygieneartikels, dahin, daß die obere dieser Lagen aus Fasern mit einer Feinheit von höchstens 3,5 dtex besteht, während die untere dieser Lagen Bikomponentenfasern mit einer Feinheit zwischen 4 und 10 dtex umfaßt, deren höherschmelzende Komponente von PES (Polyester) gebildet ist, wird einerseits - wie eingangs im Zusammenhang mit dem erfindungsgemäßen Verbundwerkstoff diskutiert -, ein angenehmes Traggefühl aufgrund der sehr feinen Fasern in der hautberührenden Lage der Schicht vermittelt und andererseits wird ein guter Faserzusammenhalt mit gutem bauschelastischem Verhalten in der unteren Lage und absolute Dichtheit gegen ein Austreten von superabsorbierenden Partikelmaterialien erreicht.

In weiterer Ausbildung der Erfindung weist der Speicherkörper außerdem eine Schicht aus nichtvernetzten Zellulosefasern mit einem Flüssigkeitsretentionswert zwischen 1,0 und 1,4 g_{F1}/g_{Faser} und wenigstens 20 Gew.-%, vorzugsweise wenigstens 40 Gew.-% superabsorbierenden Polymermaterialien auf. Diese Schicht befindet sich dann unterhalb, also körperabgewandt, von der aus intravernetzten Zellulosefasern gebildeten Schicht des Speicherkörpers.

Ferner hat es sich als vorteilhaft erwiesen, wenn die vorstehend erwähnte weitere Schicht des Speicherkörpers ihrerseits zweischichtig aufgebaut ist, indem sie auf ihrer im Gebrauch körperabgewandten Seite eine von superabsorbierenden Materialien im wesentlichen freie Schicht aufweist. Diese im wesentlichen SAP-freie Schicht, die höchstens 20 Gew.-%, vorzugsweise höchstens 10 Gew.-% an superabsorbierenden Materialien aufweist, wirkt dann quasi als Sperrmittel für körnige superabsorbierende Partikelmaterialien, die in sehr viel höherer Konzentration in der vorstehend erwähnten Speicherschicht des Speicherkörpers enthalten sind, in Richtung auf die körperabgewandte Seite, wo die Gefahr besteht, daß diese mitunter spitzen Körner die körperabgewandte flüssigkeitsundurchlässige Schicht (das Backsheet) beschädigen können.

Weitere Merkmale, Einzelheiten und Vorteile der Erfindung ergeben sich aus den beigefügten Patentansprüchen und der zeichnerischen Darstellung und nachfolgenden Beschreibung bevorzugter Ausführungsformen der Erfindung.

In der Zeichnung zeigt:
- Figur 1: eine schematische Schnittdarstellung eines erfindungsgemäßen zweilagigen Verbundwerkstoffs;
- Figur 1a: ein Prägemuster des Verbundwerkstoffs;
- Figur 2: eine schematische Schnittdarstellung eines erfindungsgemäßen Hygieneartikels.

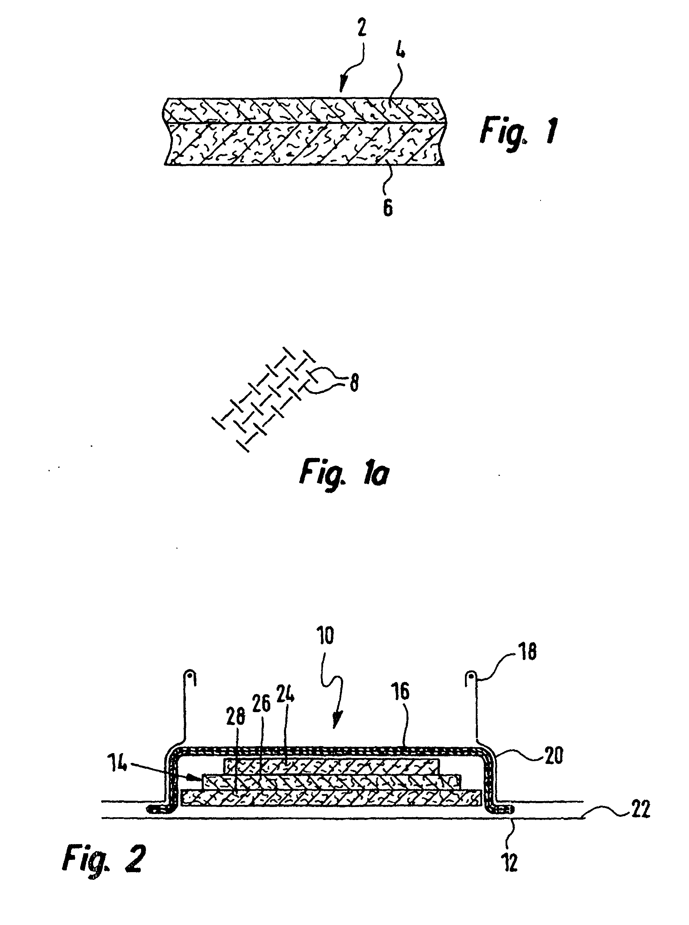

Figur 1 zeigt in schematischer Darstellung einen erfindungsgemäßen Verbundwerkstoff, der als körperzugewandte Lage in einem Hygieneartikel eingesetzt werden kann. Der Verbundwerkstoff 2 umfaßt eine erste im Gebrauch des Hygieneartikels körperzugewandte obere Lage 4 und eine zweite im Gebrauch des Hygieneartikels körperabgewandte untere Lage 6. Die obere Lage 4, die ein Flächengewicht von 18 g/m² aufweist, ist gebildet von einem Karden-Vlies aus einer Fasermischung, die zu 60 Gew.-% aus Polypropylenmonokomponentenfasern mit einer Feinheit von 2,2 dtex und zu 40 Gew.-% aus Polypropylen/Polyäthylen-Bikomponentenfasern mit einer Feinheit von 1,7 dtex gebildet ist. Diese Lage wurde kalandriert, wobei ein sogenanntes "Tric-Trac-Prägemuster" gebildet wurde, welches alternierend zueinander versetzte und in senkrechten Richtungen zueinander ausgerichtete Linienabschnitte 8 aufweist. Der Anteil der geprägten Linienabschnitte 8 an der Gesamtfläche beträgt ca. 20 %.

Der Schmelzpunkt der PP-Monokomponentenfasern liegt mit etwa 160°C höher als derjenige der niedrigschmelzenden Komponente PE der PP/PE-Bikomponentenfasern mit 110°C. Der Schmelzpunkt der höherschmelzenden PP-Komponente der PP/PE-Bikomponentenfasern beträgt etwa 140°C.

Die untere Lage 6, die ein Flächengewicht von 12 g/m² aufweist, umfaßt einen hohen Anteil von PES/PE-Bikomponentenfasern, der bei der bevorzugten Ausführungsform zu 100 % gewählt wurde. Die Bikomponentenfasern haben eine Feinheit oder Stärke von 4,4 dtex. Der Schmelzpunkt der höherschmelzenden Komponente PES (Polyester) der PES/PE-Bikomponentenfasern liegt bei ca. 260°C. Die Bikomponentenfaser ist als Kern/Mantel-Faser mit zur Längsmittelrichtung der Faser symmetrischem Kern aus PES ausgebildet.

Als besonders geeignet erweist sich eine Bikomponentenfaser mit einer Stärke von 6,7 dtex, die vorzugsweise als Kern/Mantel-Faser mit asymmetrischem Kern (höhere Spiralisierung, resiliency) ausgebildet ist.

Die untere Lage 6 wurde als kardiertes Vlies ausgebildet. Die zuvor kalandrierte obere Lage 4 und die untere Lage 6 werden übereinander angeordnet und im "Air-Through-Verfahren" durch thermische Einwirkung heißer Luft miteinander verbunden,
wobei die niedrigerschmelzende Komponente der Bikomponentenfasern (jeweils PE) durch thermische Einwirkung zumindest angeschmolzen wird und so die Fasern der einzelnen Schichten untereinander, aber auch die Fasern in einem Grenzflächenbereich zwischen den beiden Vlieslagen miteinander verbindet.

Figur 2 zeigt in schematischer Darstellung eine bevorzugte Ausführungsform eines erfindungsgemäßen Hygieneartikels 10 mit flüssigkeitsundurchlässiger körperabgewandter Folienschicht 12, einem dreischichtigen Saugkörper 14 und einer den Saugkörper auf der körper zugewandten Seite überfangenden Topsheet-Schicht 16, die ihrerseits wenigstens zweilagig ausgebildet ist. Zusätzlich umfaßt der Hygieneartikel beidseits des Saugkörpers vorgesehene und mit Elastifizierungsmitteln 18 ausgestattete Bündchenelemente 20, welche nach den aus der Darstellung ersichtlichen Seiten hin die Topsheet-Schicht überfangen und kantenbündig bis zu einem Längsrand 22 der Folienschicht 12 verlaufen.

Der Speicherkörper 14 umfaßt eine unmittelbar unterhalb der Topsheet-Schicht 16 vorgesehene und mit dieser in Kontakt stehende Schicht 24, welche aus vernetzten Zellulosefasern mit einem Anteil von 8 bis 15 Gew.-% bezogen auf das Gewicht dieser Schicht superabsorbierender Materialien besteht. Unterhalb dieser ersten Schicht 24 befindet sich eine zweite Speicherkörperschicht 26, die im wesentlichen aus nichtvernetzten Zellulosefasern mit einem Anteil von mehr als 20 Gew.-% superabsorbierenden Materialien besteht. An diese Schicht 26 schließt sich körperabgewandt eine dritte Schicht 28 an, bei der es sich auch um eine Teilschicht der zweiten Schicht 26 handeln kann, die ebenfalls aus natürlichen nichtvernetzten Zellulosefasern besteht, die jedoch keine superabsorbierenden Materialien aufweist. Diese Teilschicht dient in erster Linie als Sperrschicht für körnige superabsorbierende Partikelmaterialien und hindert diese daran, weiter nach unten in Richtung auf die Folienschicht 12 abzusacken und diese zu beschädigen.

Die körperzugewandte Topsheet-Schicht 16 ist wie bereits erwähnt zweilagig ausgebildet, wobei die in unmittelbarem Körperkontakt mit einem Benutzer des Hygieneartikels stehende obere Lage aus Fasern mit einer Feinheit von höchstens 3,5 dtex besteht, während die untere dieser Lagen Bikomponentenfasern mit einer Feinheit zwischen 4 und 10 dtex umfaßt, deren höherschmelzende Komponente von PES (Polester) gebildet ist. Diese Topsheet-Schicht 16 ist vorzugsweise wie der Verbundwerkstoff nach Figur 1 ausgebildet.

## Patentansprüche

1. Verbundwerkstoff zur Bildung einer körperzugewandten Lage bei einem absorbiernden Hygieneartikel zum einmaligen Gebrauch, aus wenigstens zwei durch thermische Einwirkung miteinander verbundenen Vlieslagen (4, 6), wobei die obere körperzugewandte Lage (4) aus einer Mischung von Monokomponentenfasern und Bikomponentenfasern gebildet ist und der Anteil der Bikomponentenfasern 30-70 Gew.-% der oberen Lage beträgt, und wobei die Feinheit der Fasern der oberen Lage höchstens 3,5 dtex beträgt, und **dadurch gekennzeichnet, daß** die untere Lage (6) zu wenigstens 40 Gew.-% Bikomponentenfasern umfasst, deren höherschmelzende Komponente von PES (Polyester) gebildet ist und deren niedrigerschmelzende Komponente einen niedrigeren Schmelzpunkt aufweist als der der Monokomponentenfasern der oberen Lage, und wobei die Feinheit der Bikomponentenfasern der unteren Lage zwischen 4 und 10 dtex beträgt.

2. Verbundwerkstoff nach Anspruch 1, **dadurch gekennzeichnet, daß** die obere körperzugewandte Lage (4) ein durch Kalandrieren erzeugtes Prägemuster aufweist, wobei der Anteil der geprägten Oberfläche 5 bis 30 % der Gesamtfläche beträgt.

3. Verbundwerkstoff nach Anspruch 2, **dadurch gekennzeichnet, daß** der Anteil der geprägten Fläche 15 bis 25 % der Gesamtfläche beträgt.

4. Verbundwerkstoff nach einem der vorstehenden Ansprüche, **dadurch gekennzeichnet, daß** das Flächengewicht der oberen Lage (4) 10 bis 30 g/m² beträgt.

5. Verbundwerkstoff nach Anspruch 4, **dadurch gekennzeichnet, daß** das Flächengewicht der oberen Lage (4) 15 bis 20 g/m² beträgt.

6. Verbundwerkstoff nach einem der vorstehenden Ansprüche, **dadurch gekennzeichnet, daß** die Fasern der oberen Lage (4) hydrophil oder permant hydrophil aviviert sind.

7. Verbundwerkstoff nach einem der vorstehenden Ansprüche, **dadurch gekennzeichnet, daß** die untere Lage (6) zu wenigstens 60 Gew.-% Bikomponentenfasern umfaßt, deren höherschmelzende Komponente von PES (Polyester) gebildet ist.

8. Verbundwerkstoff nach Anspruch 7, **dadurch gekennzeichnet, daß** die untere Lage (6) zu wenigstens 80 Gew.-% Bikomponentenfasern umfaßt, deren höherschmelzende Komponente von PES (Polyester) gebildet ist.

9. Verbundwerkstoff nach Anspruch 8, **dadurch gekennzeichnet, daß** die untere Lage (6) zu 100 % aus Bikomponentenfasern besteht, deren höherschmelzende Komponente von PES (Polyester) gebildet ist.

10. Verbundwerkstoff nach einem der vorstehenden Ansprüche, **dadurch gekennzeichnet, daß** die Bikomponentenfasern der unteren Lage (6) mit PES (Polyester) als höherschmelzende Komponente eine Kernmantelfaser ist.

11. Verbundwerkstoff nach Anspruch 10, **dadurch gekennzeichnet, daß** die Kernmantelfaser einen zur Längsmittelrichtung der Faser exzentrisch angeordneten Kern aufweist.

12. Verbundwerkstoff nach Anspruch 11, **dadurch gekennzeichnet, daß** die Feinheit der Kernmantelfaser 5 bis 8 dtex beträgt.

13. Verbundwerkstoff nach Anspruch 12, **dadurch gekennzeichnet, daß** die Feinheit der Kernmantelfaser 6 bis 7 dtex beträgt.

14. Verbundwerkstoff nach einem der vorstehenden Ansprüche, **dadurch gekennzeichnet, daß** die niedriger schmelzende Komponente der zu wenigstens 40 Gew.-% in der unteren Lage vorhandenen Bikomponentenfaser von PE (Polyäthylen) gebildet ist.

15. Absorbierender Hygieneartikel mit einer flüssigkeitsdichten im Gebrauch körperabgewandten Schicht (12), einem Speicherkörper (14) und einer flüssigkeitsdurchlässigen auf der körperzugewandten Seite des Speicherkörpers (14) vorgesehenen Schicht (16), **dadurch gekennzeichnet, dass** die flüssigkeitsdurchlässige auf der körperzugewandten Seite des Speicherkörpers (14) vorgesehene Schicht (16) einen Verbundwerkstoff nach einem oder mehreren der vorstehenden Ansprüche umfasst oder von einem solchen Verbundwerkstoff gebildet ist.

16. Absorbierender Hygieneartikel mit einer flüssigkeitsdichten im Gebrauch körperabgewandten Schicht (12), einem Speicherkörper (14) und einer flüssigkeitsdurchlässigen auf der körperzugewandten Seite des Speicherkörpers (14) vorgesehenen Schicht (16), wobei der Speicherkörper (14) eine Lage (24) aus intravernetzten Zellulosefasern mit einem Flüssigkeitsretentionswert umfasst, der aus dem Quotienten der Masse (g_{F1}) der aufgenommenen oder gebundenen Flüssigkeit und der Trockenmasse (g_{Faser}) der Zellulosefasern gebildet ist und 0,6 - 0,9 g_{F1}/g_{Faser} beträgt, wobei die Lage (24) intravernetzter Zellulosefasern zu 8-15 Gew.-% superabsorbierende Polymermaterialien enthält, wobei die flüssigkeitsdurchlässige auf der körperzugewandten Seite des Speicherkörpers (14) vorgesehene Schicht (16) ihrerseits wenigstens zweilagig ist und die obere dieser Lagen aus Fasern mit einer Feinheit von höchstens 3,5 dtex besteht während die untere dieser Lagen Bikomponentenfasern mit einer Feinheit zwischen 4 und 10 dtex umfasst deren höherschmelzende Komponente von PES gebildet ist.

17. Hygieneartikel nach Anspruch 16, **dadurch gekennzeichnet, daß** der Speicherkörper (14) außerdem eine Schicht (26) aus nicht vernetzten Zellulosefasern mit einem Flüssigkeitsretentionswert, der aus dem Quotienten der Masse (g_{F1}) der aufgenommenen oder gebundenen Flüssigkeit und der Trockenmasse (g_{Faser}) der Zellulosefasern gebildet ist und 1,0 - 1,4 g_{F1}/g_{Faser} beträgt, und wenigstens 20 Gew.-% superabsorbierenden Polymermaterialien aufweist.

18. Hygieneartikel nach Anspruch 17, **dadurch gekennzeichnet, daß** die weitere Schicht (26) des Speicherkörpers unterhalb der Schicht (24) aus intravernetzten Zellulosefasern angeordnet ist.

19. Hygieneartikel nach Anspruch 18, **dadurch gekennzeichnet, daß** die weitere Schicht (26) auf ihrer im Gebrauch körperabgewandten Seite einen von superabsorbierenden Materialien freien schichtförmigen Bereich (28) aufweist.

20. Hygieneartikel nach einem der Ansprüche 16 bis 19, **dadurch gekennzeichnet, daß** die flüssigkeitsdurchlässige auf der körperzugewandten Seite des Speicherkörpers vorgesehene Schicht (16) nach einem oder mehreren der Ansprüche 1 bis 14 ausgebildet ist.

## Claims

1. Composite material for producing a ply, which faces the body, in a disposable absorbent hygiene article, consisting of at least two non-woven plies (4, 6) connected to one another by a thermal process, the upper ply (4) facing the body being formed from a mixture of monocomponent fibres and bicomponent fibres and the content of bicomponent fibres being 30 to 70% by weight of the upper ply and the denier of the fibres of the upper ply being at most 3.5 dtex, **characterised in that** the lower ply (6) comprises at least 40% by weight bicomponent fibres, of which the higher-melting component is formed by PES (polyester) and of which the lower-melting component has a lower melting point than that of the monocomponent fibres of the upper ply, the denier of the bicomponent fibres of the lower ply being between 4 and 10 dtex.

2. Composite material according to claim 1, **characterised in that** the upper ply (4) facing the body comprises an embossed pattern produced by calendering, the embossed surface making up 5 to 30% of the overall surface.

3. Composite material according to claim 2, **characterised in that** embossed surface makes up 15 to 25% of the overall surface.

4. Composite material according to any of the preceding claims, **characterised in that** the G.S.M. of the upper ply (4) is 10 to 30 g/m².

5. Composite material according to claim 4, **characterised in that** the G.S.M. of the upper ply (4) is 15 to 20 g/m².

6. Composite material according to any of the preceding claims, **characterised in that** the fibres of the upper ply (4) are hydrophilically or permanently hydrophilically brightened.

7. Composite material according to any of the preceding claims, **characterised in that** the lower ply (6) comprises at least 60% by weight bicomponent fibres, of which the higher-melting component is formed by PES (polyester).

8. Composite material according to claim 7, **characterised in that** the lower ply (6) comprises at least 80% by weight bicomponent fibres, of which the higher-melting component is formed by PES (polyester).

9. Composite material according to claim 8, **characterised in that** the lower ply (6) consists of 100% bicomponent fibres, of which the higher-melting component is formed by PES (polyester).

10. Composite material according to any of the preceding claims, **characterised in that** the bicomponent fibres of the lower ply (6) with PES (polyester) as the higher-melting component is a core fibre.

11. Composite material according to claim 10, **characterised in that** the core fibre comprises a core arranged eccentrically with respect to the longitudinal central direction of the fibres.

12. Composite material according to claim 11, **characterised in that** the denier of the core fibre is 5 to 8 dtex.

13. Composite material according to claim 12, **characterised in that** the denier of the core fibre is 6 to 7 dtex.

14. Composite material according to any of the preceding claims, **characterised in that** the lower-melting component of the bicomponent fibres present to at least 40% by weight in the lower ply is formed by PE (polyethylene).

15. Absorbent hygiene article with a liquid-tight layer (12) facing the body in use, a storage structure (14) and a liquid-permeable layer (16) provided on the side of the storage structure (14) facing the body, **characterised in that** the liquid-permeable layer (16) provided on the side of the storage structure (14) facing the body comprises a composite material according to one or more of the preceding claims or is formed by such a composite material.

16. Absorbent hygiene article comprising a fluid-tight layer (12) facing the body in use, a storage structure (14) and a liquid-permeable layer (16) provided on the side of the storage structure (14) facing the body, wherein the storage structure (14) comprises a ply (24) made of intracrosslinked cellulose fibres with a liquid retention value formed from the quotient of the mass (g_{F1}) of the absorbed or bound liquid and the dry mass (g_{fibre}) of the cellulose fibres and is 0.6 to 0.9 g_{F1}/g_{fibre}, wherein the ply (24) of intracrosslinked cellulose fibres contains 8 to 15% by weight superabsorbent polymer materials, wherein the liquid-permeable layer (16) provided on the side of the storage structure (14) facing the body is in turn at least two-ply and the upper of these plies consists of fibres with a denier of at most 3.5 dtex, while the lower of these plies comprises bicomponent fibres with a denier between 4 and 10 dtex, of which the higher-melting component is formed by PES.

17. Hygiene article according to claim 16, **characterised in that** the storage structure (14) also comprises a layer (26) of non-crosslinked cellulose fibres with a liquid retention value formed from the quotient of the mass (g_{F1}) of the absorbed or bound liquid and of the dry mass (g_{fibre}) of the cellulose fibres and is 1.0 to 1.4 g_{F1}/g_{fibre}, and comprises at least 20% by weight superabsorbent polymer materials.

18. Hygiene article according to claim 17, **characterised in that** the further layer (26) of the storage structure is arranged beneath the layer (24) made of intracrosslinked cellulose fibres.

19. Hygiene article according to claim 18, **characterised in that** the further layer (26), on its side remote from the body in use, comprises a layered region (28) free from superabsorbent materials.

20. Hygiene article according to any of claims 16 to 19, **characterised in that** the liquid-permeable layer (16) provided on the side of the storage structure facing the body is constructed according to one or more of claims 1 to 14.

## Revendications

1. Matériau composite servant à former une couche, orientée vers le corps, d'un article d'hygiène absorbant à usage unique, constitué d'au moins deux couches de non-tissé (4, 6) reliées entre elles par action thermique, la couche supérieure (4) orientée vers le corps étant formée d'un mélange de fibres à un composant et de fibres à deux composants et la part des fibres à deux composants représentant 30 à 70 % en poids de la couche supérieure, et la finesse des fibres de la couche supérieure étant de 3,5 dtex maximum,
et **caractérisé en ce que** la couche inférieure (6) comprend au moins 40 % en poids de fibres à deux composants, dont le composant ayant le point de fusion le plus élevé est formé par du PES (polyester) et dont le composant ayant le point de fusion le plus bas présente un point de fusion inférieur à celui des fibres à un composant de la couche supérieure, et la finesse des fibres à deux composants de la couche inférieure étant comprise entre 4 et 10 dtex.

2. Matériau composite selon la revendication 1,
**caractérisé en ce que** la couche supérieure (4) orientée vers le corps présente un motif gaufré produit par calandrage, la part de la surface gaufrée représentant 5 à 30 % de la surface totale.

3. Matériau composite selon la revendication 2,
**caractérisé en ce que** la part de la surface gaufrée représente 15 à 25 % de la surface totale.

4. Matériau composite selon l'une quelconque des revendications précédentes,
**caractérisé en ce que** le poids par unité de surface de la couche supérieure (4) est compris entre 10 et 30 g/m².

5. Matériau composite selon la revendication 4,
**caractérisé en ce que** le poids par unité de surface de la couche supérieure (4) est compris entre 15 et 20 g/m².

6. Matériau composite selon l'une quelconque des revendications précédentes,
**caractérisé en ce que** les fibres de la couche supérieure (4) sont avivées de manière hydrophile ou hydrophile permanente.

7. Matériau composite selon l'une quelconque des revendications précédentes,
**caractérisé en ce que** la couche inférieure (6) comprend au moins 60 % en poids de fibres à deux composants dont le composant ayant le point de fusion le plus élevé est formé par du PES (polyester).

8. Matériau composite selon la revendication 7,
**caractérisé en ce que** la couche inférieure (6) comprend au moins 80 % en poids de fibres à deux composants dont le composant ayant le point de fusion le plus élevé est formé par du PES (polyester).

9. Matériau composite selon la revendication 8,
**caractérisé en ce que** la couche inférieure (6) est constituée à 100 % de fibres à deux composants dont le composant ayant le point de fusion le plus élevé est formé par du PES (polyester).

10. Matériau composite selon l'une quelconque des revendications précédentes,
**caractérisé en ce que** les fibres à deux composants de la couche inférieure (6) avec le PES (polyester) comme composant ayant le point de fusion le plus élevé est une fibre à saut d'indice.

11. Matériau composite selon la revendication 10,
**caractérisé en ce que** la fibre à saut d'indice présente un noyau agencé de manière excentrée par rapport à la direction médiane longitudinale de la fibre.

12. Matériau composite selon la revendication 11,
**caractérisé en ce que** la finesse de la fibre à saut d'indice est de 5 à 8 dtex.

13. Matériau composite selon la revendication 12,
**caractérisé en ce que** la finesse de la fibre à saut d'indice est de 6 à 7 dtex.

14. Matériau composite selon l'une quelconque des revendications précédentes,
**caractérisé en ce que** le composant ayant le point de fusion le plus bas de la fibre à deux composants présente au moins à 40 % en poids dans la couche inférieure est formé par du PE (polyéthylène).

15. Article d'hygiène absorbant comprenant une couche (12) imperméable aux liquides opposée au corps pendant l'usage, un corps accumulateur (14) et une couche (16) perméable aux liquides prévue sur la face du corps accumulateur (14) orientée vers le corps,
**caractérisé en ce que** la couche (16) perméable aux liquides prévue sur la face du corps accumulateur (14) orientée vers le corps comprend un matériau composite selon l'une ou plusieurs des revendications précédentes ou est formée par un tel matériau composite.

16. Article d'hygiène absorbant comportant une couche (12) imperméable aux liquides opposée au corps pendant l'usage, un corps accumulateur (14) et une couche (16) perméable aux liquides prévue sur la face du corps accumulateur (14) orientée vers le corps, le corps accumulateur (14) comprenant une couche (24) en fibres de cellulose intraréticulées ayant un coefficient de rétention de liquide qui est formé par le quotient de la masse (g_{F1}) du liquide absorbé ou lié et de la masse sèche (g_{fibre}) des fibres de cellulose et est compris entre 0,6 et 0, 9 g_{F1}/g_{fibre}, la couche (24) de fibres de cellulose intraréticulées contenant de 8 à 15 % en poids de matériaux polymères superabsorbants, la couche (16) perméable aux liquides prévue sur la face du corps accumulateur (14) orientée vers le corps comprenant à son tour au moins deux couches et la couche supérieure de ces deux couches étant constituée de fibres d'une finesse de 3,5 dtex maximum, tandis que la couche inférieure de ces couches comprend des fibres à deux composants d'une finesse comprise entre 4 et 10 dtex, dont le composant ayant le point de fusion le plus élevé est formé par du PES.

17. Article d'hygiène selon la revendication 16,
**caractérisé en ce que** le corps accumulateur (14) présente en outre une couche (26) constituée de fibres de cellulose non réticulées ayant un coefficient de rétention de liquide qui est formé par le quotient de la masse (g_{F1}) du liquide absorbé ou lié et de la masse sèche (g_{fibre}) des fibres de cellulose et est compris entre 1,0 et 1,4 g_{F1}/g_{fibre}, et présente au moins 20 % en poids de matériaux polymères superabsorbants.

18. Article d'hygiène selon la revendication 17,
**caractérisé en ce que** l'autre couche (26) du corps accumulateur est agencée au-dessous de la couche (24) de fibres de cellulose intraréticulées.

19. Article d'hygiène selon la revendication 18,
**caractérisé en ce que** l'autre couche (26) présente, sur sa face opposée au corps pendant l'usage, une zone disposée en couches (28) exempte de matériaux superabsorbants.

20. Article d'hygiène selon l'une des revendications 16 à 19,
**caractérisé en ce que** la couche (16) perméable aux liquides prévue sur la face du corps accumulateur orientée vers le corps est réalisée selon l'une ou plusieurs des revendications 1 à 14.
